# EUROPÄISCHE PATENTANMELDUNG

(11) **EP 2 229 886 A1**
(43) Veröffentlichungstag der Anmeldung: **22.09.2010**
(21) Anmeldenummer: 09003796.1
(22) Anmeldetag: 17.03.2009
(51) Int. Cl.: A61B 5/15

(54) **Lanzette mit Stechelement aus Kunststoff**

(71) Anmelder: Roche Diagnostics GmbH, 68305 Mannheim (DE); F. Hoffmann-La Roche AG, 4070 Basel (CH)
(72) Erfinder: Keil, Michael, 67071 Ludwigshafen (DE); Seul, Thomas, Prof. Dr., 98574 Schmalkalden (DE); Reinhold, Thomas Sowden, 48161 Münster (DE); Schatte, Verena, 32049 Herford (DE); Eilers, Rolf, 32549 Bad Oeynhausen (DE)
(74) Vertreter: Twelmeier Mommer & Partner

(57) **Zusammenfassung**

Die Erfindung betrifft eine Lanzette mit einem Stechelement (1), das zumindest teilweise aus Kunststoff besteht. Erfindungsgemäß ist vorgesehen, dass das Stechelement (1) mit einer Sicke (5) versteift ist. Die Erfindung betrifft ferner ein Verfahren zum Herstellen eines Stechelements (1), wobei in einen Kunststoffstreifen eine Sicke (5) eingeprägt und eine Spitze erzeugt wird.

## Beschreibung

Als Material für Stechelemente von Lanzetten wird üblicher Weise Stahl verwendet. Als Alternativen wurden in der Literatur unter anderem Kunststoffe, Faserverbundwerkstoffe, Glas und andere keramische Materialien vorgeschlagen, beispielsweise in der US 2006/0100542 A9. Gegenüber Stahl haben alle diese Materialien jedoch erhebliche Nachteile, die eine praktische Realisierung bisher verhindert haben, obwohl alternative Werkstoffe gegenüber Stahl ein erhebliches Potential zur Einsparung von Herstellungskosten bieten.

Kunststoffe sind im Allgemeinen zu weich für die Herstellung von kleinen Stechelementen, die einen schmerzarmen Einstich ermöglichen sollen. Gefüllte Kunststoffe, die in der englischsprachigen Literatur als Composits bezeichnet werden, haben in dieser Hinsicht etwas bessere Eigenschaften, lösen die mit Kunststoffen verbundenen Materialprobleme jedoch allenfalls zum Teil. Keramische Werkstoffe haben zwar eine sehr große Härte, sind jedoch in der Regel auch spröde und brechen leicht, so dass die Gefahr besteht, dass bei Gebrauch Splitter oder Bruchstücke eines Stechelements in einer Stichwunde bleiben. Dieses Problem tritt sowohl bei Gläsern als auch bei kristallinen Materialien auf.

Angesichts dieser Probleme ist es Aufgabe der vorliegenden Erfindung, einen Weg aufzuzeigen, wie eine Lanzette mit einem nichtmetallischen Stechelement verwirklicht werden kann.

Diese Aufgabe wird erfindungsgemäß durch eine Lanzette mit den im Anspruch 1 angegebenen Merkmalen gelöst. Vorteilhafte Weiterbildungen der Erfindung sind Gegenstand von Unteransprüchen.

Eine erfindungsgemäße Lanzette hat ein zumindest teilweise, bevorzugt überwiegend, aus Kunststoff bestehendes Stechelement, das mit einer Sicke versteift ist. Mittels einer Sicke, die auf einer Seite eines Stechelements eine rinnenartige Vertiefung und auf der gegenüberliegenden Seite eine längliche Erhöhung bildet, lässt sich selbst bei dünnen Stechelementen mit einer Materialstärke im Bereich von Bruchteilen eines Millimeters und einer Breite von beispielsweise weniger als einem Millimeter die Formstabilität soweit erhöhen, wie es für einen schmerzarmen Einstich erforderlich ist. Insbesondere kann durch eine Sicke selbst bei schmalen Stechelementen aus Kunststoff die Biegesteifigkeit soweit erhöht werden, dass sich das Stechelement beim Eindringen in die Haut eines Patienten nicht biegt.

Die Sicke erstreckt sich bevorzugt in Längsrichtung des Stechelements, beispielsweise parallel zur Stichrichtung. Auf diese Weise kann die Formstabilität eines schmalen Stechelements aus Kunststoff besonders wirksam erhöht werden. Prinzipiell kann ein Stechelement auch mehrere Sicken aufweisen. Bevorzugt hat es jedoch nur eine einzige Sicke.

Eine weitere vorteilhafte Weiterbildung der Erfindung sieht vor, dass die Sicke eine an der Spitze des Stechelements offene Rinne ist. Blickt man genau entgegengesetzt zur Stichrichtung auf die Spitze einer solchen Lanzette kann man also in die Rinne hinein sehen. Eine derartige Sicke hat den Vorteil, die Formstabilität des Stechelements insbesondere auch im Bereich der für einen schmerzarmen Einstich besonders wichtigen Spitze zu erhöhen.

Bevorzugt ist die Sicke in das Stechelement eingeprägt. Möglich ist es jedoch auch, das Stechelement beispielsweise durch Spritzgießen unmittelbar mit einer Sicke herzustellen. Das Einprägen einer Sicke verursacht als zusätzlicher Arbeitsschritt zwar einen erhöhten Fertigungsaufwand, überraschender Weise lassen sich die Materialeigenschaften eines Kunststoffs durch den Prägeschritt verbessern, so dass ein Stechelement mit einer eingeprägten Sicke eine besonders hohe Formstabilität aufweist.

Eine weitere vorteilhafte Weiterbildung der Erfindung sieht vor, dass das Stechelement eine laserstrahlgeschnittene Spitze aufweist. Beim Laserstrahlschneiden kann vorteilhaft ein besonders harter Rand und damit eine besonders scharfe Spitze oder Klinge erzeugt werden. Die Einwirkung eines Laserstrahls kann dabei die Materialeigenschaften an der Oberfläche verändern und eine Härtung bewirken. Besonders bevorzugt ist dabei, das Laserstrahlschneiden erst nach dem Einprägen der Sicke vorzunehmen.

Die Erfindung betrifft ferner ein Verfahren zum Herstellen eines Stechelements aus Kunststoff, wobei in einen Kunststoffstreifen eine Sicke eingeprägt und nach dem Einprägen eine Spitze erzeugt wird. Bevorzugt wird die Spitze mittels laserstrahlschneiden erzeugt. Die Spitze kann beispielsweise auch durch Stanzen erzeugt werden.

Die vorliegende Erfindung betrifft ferner eine Lanzette mit einem Stechelement, das aus einem Werkstoffverbund besteht, der eine Kunststoffschicht und eine Keramikschicht aufweist. Diese Maßnahe hat den Vorteil, dass bei einem Bruch der Keramikschicht die Bruchstücke an der Kunststoffschicht haften bleiben. Die Gefahr, dass Bruchstücke bei einem Stich im Körper eines Patienten zurück bleiben, lässt sich auf diese Weise deutlich reduzieren. Bevorzugt ist die Keramikschicht zwischen zwei Kunststoffschichten angeordnet. Ein derartiges Stechelement kann also als Schichtverbund ausgebildet sein. Besonders bevorzugt ist dabei, dass die Keramikschicht dicker als die eine oder die beiden Kunststoffschichten ist.

Ein derartiges Stechelement in Sandwichbauweise kann flach ausgebildet sein oder eine versteifende Sicke aufweisen. Die keramische Schicht kann beispielsweise aus Glas, insbesondere einem Borosilikatglas, oder einem kristallinen Material bestehen.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen bezeichnet.

### Es zeigen:

- Figur 1: eine schematische Darstellung eines Ausführungsbeispiels einer erfindungsgemäßen Lanzette;
- Figur 2: eine Schnittansicht zu Figur 1 entlang der Schnittlinie AA;
- Figur 3: eine schematische Darstellung eines weiteren Ausführungsbeispiels einer erfindungsgemäßen Lanzette;
- Figur 4: eine Schnittansicht zu Figur 3 entlang der Schnittlinie AA;
- Figur 5: eine schematische Darstellung eines Längsschnitts durch ein weiteres Ausführungsbeispiel eines Stechelements.

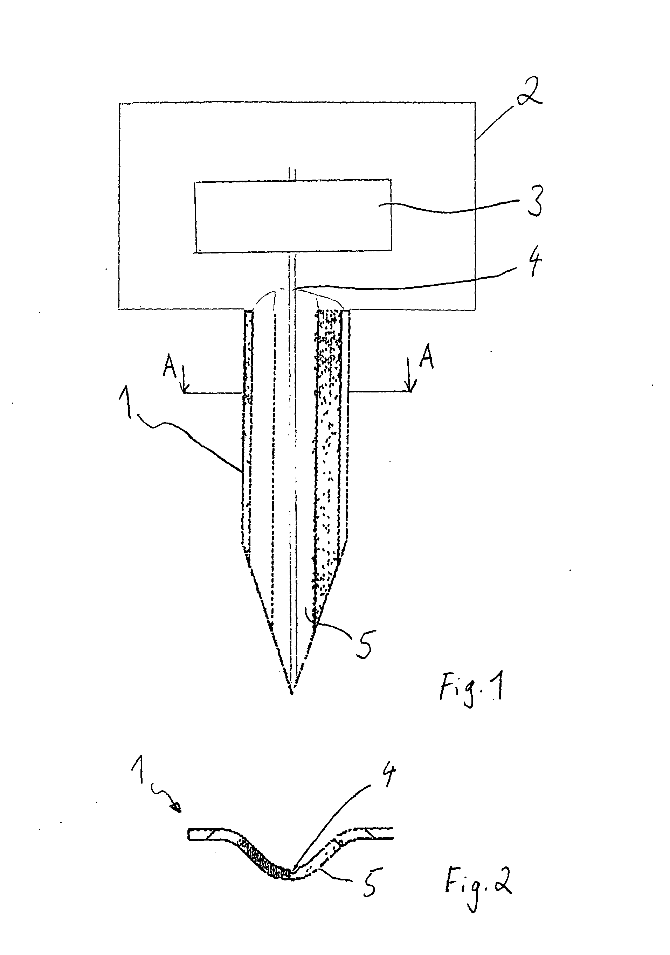

Die in Figur 1 dargestellte Lanzette hat ein Kunststoffstechelement 1 zum Einstechen in die Haut eines Patienten. Das Stechelement 1 geht bei dem dargestellten Ausführungsbeispiel von einem Grundkörper 2 aus, der eine größere Breite als das Stechelement 1 hat. Auf dem Grundkörper 2 ist ein Testbereich 3, beispielsweise in Form eines aufgeklebten Testfeldes mit Nachweisreagenzien zur Bestimmung einer Analytkonzentration, beispielsweise der Glucosekonzentration, angeordnet. Dem Testbereich 3 kann eine Körperflüssigkeitsprobe mittels eines Kapillarkanals 4 zugeführt werden, der von einem vorderen Bereich des Stechelements 1 ausgeht, der bei einem Stich in den Körper eines Patienten eindringt. Bevorzugt geht der Kappilarkanal 4 von der Spitze aus, ist also in Stichrichtung offen.

Bei einem Stich kann die Lanzette über den Grundkörper 2 an ein Stechgerät ankoppeln. Prinzipiell ist es jedoch auch möglich, eine Lanzette ohne einen Grundkörper 2 auszubilden, also eine Lanzette als ein Stechelement 1 ohne zusätzliche Teile auszubilden. Eine Lanzette kann mit oder ohne Grundkörper 2 auf einem Trägerband angeordnet werden und bei einem Stich beispielsweise zusammen mit dem Trägerband bewegt werden, wie dies in der EP 1 943 948 A1 beschrieben ist, auf die diesbezüglich verweisen wird.

Das Stechelement 1 besteht aus Kunststoff und ist mit einer Sicke 5 versteift. Die Sicke 5 erstreckt sich in Längsrichtung des Stechelements 1 und ist auf einer Oberseite des Stechelements 1 als eine in der Spitze des Stechelements 1 offene Rille ausgebildet. Auf der Rückseite des Stechelements 1 ist die Sicke 5 eine in Längsrichtung des Stechelements 1 verlaufende Erhebung. Bei dem dargestellten Ausführungsbeispiel endet die Sicke 5 vor dem Testbereich 3. Es ist aber auch möglich, dass sich die Sicke 5 durch den Grundkörper 2 hindurch erstreckt oder bereits in einem hinteren Abschnitt des Stechelements 1 endet.

Der Kapillarkanal 4 ist bei dem dargestellten Ausführungsbeispiel auf der Innenseite der Sicke 5, d. h. in der Rinne, angeordnet, kann jedoch auch auf der Außenseite der Sicke 5, d. h. auf der Erhebung, angeordnet sein. Der Kapillarkanal 4 kann beispielsweise eingeritzt werden oder beim Einprägen der Sicke 5 mit eingeprägt werden. Von dem Kapillarkanal 4 wird Körperflüssigkeit durch Kapillarkräfte zu dem Testbereich 3 transportiert. Der Kapillarkanal 4 kann mit einem hydrophilen Material beschichtet sein, beispielsweise mit Heparin. Die Breite des Kapillarkanals 4 beträgt nur einen Bruchteil der Breite der Sicke 5, beispielsweise weniger als ein Drittel, vorzugsweise weniger als ein Fünftel. Typischerweise hat der Kapillarkanal 4 eine Breite von 50 µm bis 150 µm.

Das Stechelement 1 hat bei dem dargestellten Ausführungsbeispiel eine Breite von weniger als einem Millimeter, insbesondere eine Breite von 0,3 mm bis 0,8 mm, nämlich eine Breite von etwa 0,5 mm bis 0,7 mm. Der Grundkörper 2 und das Stechelement 1 sind dabei einstückig ausgebildet. Die dargestellte Lanzette kann hergestellt werden, indem in einen Kunststoffstreifen eine Sicke 5 eingeprägt wird. Anschließend wird das Stechelement 1 mit seiner Spitze mittels laserstrahlschneiden ausgeschnitten.

Der verwendete Kunststoff ist bevorzugt ein gefüllter Kunststoff, insbesondere ein mit Fasern gefüllter Kunststoff. Als Füllstoffe sind beispielsweise Talkum, Glas oder andere anorganische Stoffe geeignet. Besonders vorteilhaft sind als Füllstoffe Fasern, insbesondere Langfasern, geeignet, beispielsweise Glasfasern oder Kohlenstofffasern. Besonders vorteilhaft sind dabei in Längsrichtung des Stechelements 1 ausgerichtete Fasern. Es ist deshalb bevorzugt, wenn mindestens die Hälfte der Fasern in Längsrichtung des Stechelements 1 ausgerichtet sind. Fasern werden als Füllstoff bevorzugt in Form von Fasermatten verwendet. Der Anteil der Füllstoffe am Gesamtvolumen des verwendeten Kunststoffs beträgt typischerweise zwischen einem Zehntel und drei Vierteln, bevorzugt ein Fünftel bis zwei Drittel. Als Kunststoff kann beispielsweise Polyetheretherketon verwendet werden.

In Figur 2 ist ein Schnitt durch das Stechelement 1 entlang der Schnittlinie AA von Figur 1 gezeigt. Darin ist zu erkennen, dass die Sicke 5 in dem dargestellten Ausführungsbeispiel eine Tiefe hat, die größer als die Materialstärke des Stechelements 1 ist. Bevorzugt ist die Tiefe der Sicke 5 mindestens doppelt so groß wie die Materialstärke des Stechelements 1. Die Materialstärke des Stechelements 1 kann beispielsweise zwischen 0,05 mm und 0,5 mm betragen, bevorzugt 0,1 mm bis 0,3 mm.

Die Sicke 5 hat bei dem dargestellten Ausführungsbeispiel eine Breite, die etwa Zweidrittel der Breite des Stechelements 1 beträgt. Bevorzugt beträgt die Breite der Sicke 5 zwischen der Hälfte und Dreivierteln des Stechelements 1.

Wie Figur 2 zeigt hat die Sicke 5 einen bogenförmigen Querschnitt, der bevorzugt abgerundete Ecken aufweist, also an seinem Rand abgerundet ist. Es können jedoch auch spitze Sicken oder Kastensicken verwendet werden.

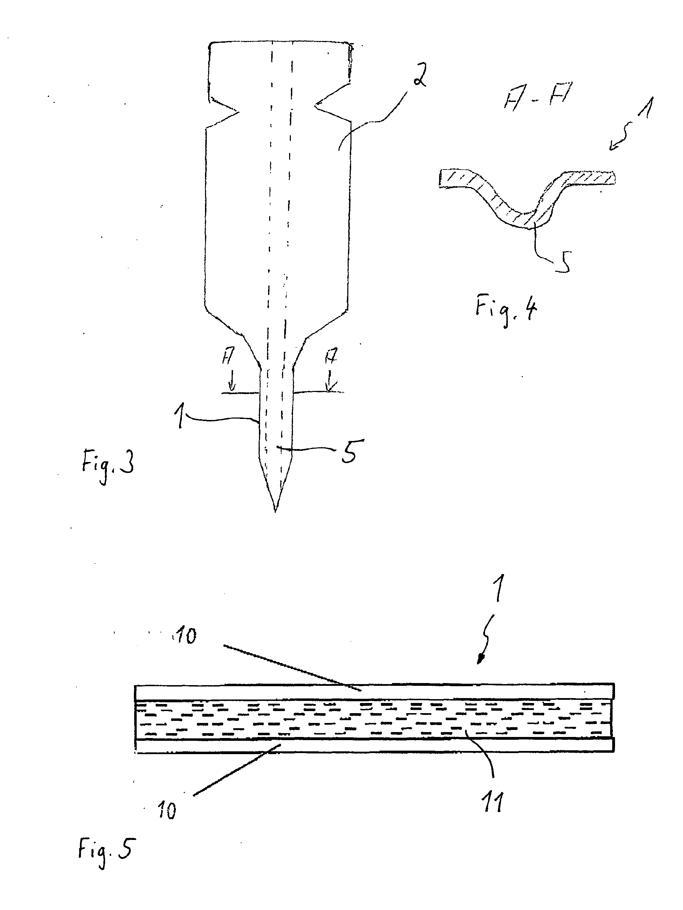

Figur 3 zeigt ein weiteres Ausführungsbeispiel einer Lanzette mit einem Stechelement 1 und einem Grundkörper 2. Im Unterschied zu dem vorstehend beschriebenen Ausführungsbeispiel ist auf dem Grundkörper 2 kein Testbereich vorgesehen und auch kein Kapillarkanal vorhanden. Die Sicke 5 geht auf voller Länge durch den Grundkörper 2 und das Stechelement 1 hindurch. Die Lanzette ist ebenso wie das vorstehend beschriebene Ausführungsbeispiel aus einem gefüllten Kunststoff.

Figur 5 zeigt ein weiteres Ausführungsbeispiel des Stechelements 1 einer Lanzette in einem Längsschnitt. Das Stechelement 1 besteht aus einem Werkstoffverbund der eine zwischen zwei Kunststoffschichten 10 angeordnete Keramikschicht 11, beispielsweise aus einem Borosilikatglas aufweist. Das in Figur 5 dargestellte Stechelement 1 ist flach, kann jedoch auch mit einer Sicke 5 wie das vorstehend beschriebene Ausführungsbeispiel ausgebildet werden. Bei einem keramischen Stechelement 1 wird die Sicke 5 bevorzugt in einen Rolling eingeprägt, der anschließend gesintert wird. Die Keramikschicht 11 ist bei dem dargestellten Ausführungsbeispiel dicker als die beiden Kunststoffschichten 10, nämlich mehr als doppelt so dick.

### Bezugszahlen

- 1: Stechelement
- 2: Grundkörper
- 3: Testbereich
- 4: Kapillarkanal
- 5: Sicke
- 10: Kunststoffschichten
- 11: Keramikschicht

## Patentansprüche

1. Lanzette mit einem Stechelement (1), das zumindest teilweise aus Kunststoff besteht, **dadurch gekennzeichnet, dass** das Stechelement (1) mit einer Sicke (5) versteift ist.

2. Lanzette nach Anspruch 1 **dadurch gekennzeichnet, dass** sich die Sicke (5) in Längsrichtung des Stechelements (1) erstreckt.

3. Lanzette nach einem der vorstehenden Ansprüche **dadurch gekennzeichnet, dass** die Sicke (5) eine an der Spitze des Stechelements (1) offene Rinne ist.

4. Lanzette nach einem der vorstehenden Ansprüche **dadurch gekennzeichnet, dass** die Sicke (5) im Querschnitt bogenförmig ist.

5. Lanzette nach einem der vorstehenden Ansprüche **dadurch gekennzeichnet,** die Sicke (5) eingeprägt ist.

6. Lanzette nach einem der vorstehenden Ansprüche, **dadurch gekennzeichnet, dass** der Kunststoff ein gefüllter Kunststoff, vorzugsweise ein mit Fasern gefüllter Kunststoff, ist.

7. Lanzette nach Anspruch 6, **dadurch gekennzeichnet, dass** mindestens die Hälfte der Fasern in Längsrichtung des Stechelements (1) ausgerichtet sind.

8. Lanzette nach einem der vorstehenden Ansprüche, **dadurch gekennzeichnet, dass** das Stechelement (1) eine laserstrahlgeschnittene Spitze aufweist.

9. Lanzette nach einem der vorstehenden Ansprüche, **dadurch gekennzeichnet, dass** in dem Stechelement (1) ein Kapillarkanal (4) verläuft.

10. Lanzette nach einem der vorstehenden Ansprüche, **dadurch gekennzeichnet, dass** der Kunststoff ein Polyetheretherketon ist.

11. Lanzette nach einem der vorstehenden Ansprüche, **dadurch gekennzeichnet, dass** das Stechelement (1) eine Breite von weniger als 1 mm, insbesondere von 0,3 mm bis 0,8 mm hat.

12. Lanzette nach einem der vorstehenden Ansprüche, **dadurch gekennzeichnet, dass** die Sicke (5) eine Tiefe hat, die größer als die Materialstärke des Stechelements (1) ist, vorzugsweise mindestens doppelt so groß wie die Materialstärke des Stechelements (1) ist.

13. Lanzette nach einem der vorstehenden Ansprüche, **dadurch gekennzeichnet, dass** die Sicke (5) eine Breite hat, die zwischen der Hälfte und drei Vierteln der Breite des Stechelements (1) beträgt.

14. Verfahren zum Herstellen eines Stechelements (1), wobei in einen Kunststoffstreifen eine Sicke (5) eingeprägt und eine Spitze erzeugt wird.

15. Lanzette mit einem Stechelement (1), das aus einem Werkstoffverbund besteht, der eine Kunststoffschicht (10) und eine Keramikschicht (11) aufweist.
